# EUROPEAN PATENT APPLICATION

(11) **EP 4 368 106 A1**
(43) Date of publication of application: **15.05.2024**
(21) Application number: 22858721.8
(22) Date of filing: 16.08.2022
(51) Int. Cl.: A61B 5/346, A61B 5/327, A61B 5/28, A61B 5/00, G16H 50/20, G16H 50/30

(54) **SYSTEM FOR PREDICTING HEALTH STATE USING SINGLE-LEAD ELECTROCARDIOGRAM DEVICE**

(30) Priority: 17.08.2021 KR 20210107768
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012215
(87) International publication number: WO 2023/022484

(57) **Abstract**

The present invention discloses a system for predicting a health condition using a single-lead electrocardiogram device, the system including: a single-lead electrocardiogram measurement unit (110) provided with two electrodes, and configured to obtain asynchronous electrocardiogram data by measuring electrocardiograms for at least two electrical axes at different times; and a prediction unit (120) configured to predict the presence/absence of a disease and the degree of the disease from the asynchronous electrocardiogram data input from the single-lead electrocardiogram measurement unit (110) through a diagnostic algorithm (121) previously constructed by being trained on a plurality of standard electrocardiogram datasets in which standard lead electrocardiograms measured for the same electrical axes are matched to the presence/absence of diseases and degrees of the diseases corresponding to the standard lead electrocardiograms.

## Description

### Technical Field

The present invention relates to a system for predicting a health condition using a single-lead electrocardiogram device that may easily measure 2- or more-lead asynchronous electrocardiograms using a single-lead electrocardiogram device or a 6-lead electrocardiogram device and may predict a disease from the measured 2- or more-lead asynchronous electrocardiogram data.

### Background Art

As is well known, since the development of an electrocardiogram, knowledge related to the electrocardiogram has expanded exponentially. Through electrocardiogram tests, information about the electrical functions of the heart can be obtained and various heart diseases such as arrhythmia, coronary artery disease, and myocardial disease can be diagnosed.

Recently, research into AI algorithms for electrocardiograms has been actively conducted. By AI algorithms, heart failure is detected, atrial fibrillation among arrhythmia rhythms is predicted, and gender is determined.

As described above, by overcoming human limitations, subtle changes in electrocardiogram waveforms can be detected and electrocardiogram interpretation can be further improved by AI algorithms.

Meanwhile, the electrocardiograms used in the medical field are 12-lead electrocardiograms. Such a 12-lead electrocardiogram is measured by attaching 10 electrodes, including 3 limb electrodes, 6 chest electrodes and 1 ground electrode, and the measured electrocardiogram data can be transmitted remotely.

However, it is inconvenient to expose the chest, attach 10 electrodes and then use a device in everyday life, so that a portable pad measurement device, a Galaxy Watch, an Apple Watch, or the like capable of measuring a single-lead electrocardiogram is also used.

A single-lead electrocardiogram measured as described above can be used to diagnose arrhythmia. However, a single-lead electrocardiogram has limitations in terms of use in the diagnosis of a disease requiring electrocardiogram information for various leads, such as myocardial infarction.

Therefore, there is a demand for technology that can easily measure 2- or more-lead asynchronous electrocardiograms using a single-lead electrocardiogram device or a 6-lead electrocardiogram device and predict a disease from the measured 2- or more-lead asynchronous electrocardiogram data.

### Disclosure

### Technical Problem

A technical object to be achieved by the spirit of the present invention is to provide a system for predicting a health condition using a single-lead electrocardiogram device that may easily measure 2- or more-lead asynchronous electrocardiograms using a single-lead electrocardiogram device or a 6-lead electrocardiogram device and may predict a disease from the measured 2- or more-lead asynchronous electrocardiogram data.

### Technical Solution

In order to accomplish the above-described object, an embodiment of the present invention provides a system for predicting a health condition using a single-lead electrocardiogram device, the system including: a single-lead electrocardiogram measurement unit provided with two electrodes, and configured to obtain asynchronous electrocardiogram data by measuring electrocardiograms for at least two electrical axes at different times; and a prediction unit configured to predict the presence/absence of a disease and the degree of the disease from the asynchronous electrocardiogram data input from the single-lead electrocardiogram measurement unit through a diagnostic algorithm previously constructed by being trained on a plurality of standard electrocardiogram datasets in which standard lead electrocardiograms measured for the same electrical axes are matched to the presence/absence of diseases and degrees of the diseases corresponding to the standard lead electrocardiograms.

In this case, the diagnostic algorithm may be constructed to output and predict the presence/absence of a disease and the degree of the disease corresponding to asynchronously measured standard lead electrocardiogram data by using the asynchronously measured standard lead electrocardiogram data as input.

Furthermore, the diagnostic algorithm may be constructed to output and predict the presence/absence of a disease and the degree of the disease corresponding to standard lead electrocardiogram data by using the standard lead electrocardiogram data, in which time series information has been deleted from synchronously measured standard lead electrocardiogram data, as input.

Furthermore, the diagnostic algorithm may be constructed to convert a plurality of pieces of asynchronous electrocardiogram data, measured and input at different times from the single-lead electrocardiogram measurement unit, into synchronous electrocardiogram data and predict the presence/absence of a disease and the degree of the disease from the resulting synchronous electrocardiogram data.

Furthermore, the system may further include an electrocardiogram data generation unit configured to generate a plurality of pieces of standard lead electrocardiogram data that are not measured by the single-lead electrocardiogram measurement unit and, thus, are not matched to standard lead electrocardiograms based on a plurality of pieces of asynchronous electrocardiogram data measured by the single-lead electrocardiogram measurement unit; and the diagnostic algorithm may output and predict the presence/absence of a disease and the degree of the disease by using the measured asynchronous electrocardiogram data and the generated standard lead electrocardiogram data as input or using the generated standard lead electrocardiogram data as input.

Furthermore, the diagnostic algorithm may be trained with the individual characteristic information of an examinee reflected therein; and the prediction unit may predict the presence/absence of a disease and the degree of the disease from the asynchronous electrocardiogram data, measured by the single-lead electrocardiogram measurement unit, with the individual characteristic information reflected therein.

Furthermore, the individual characteristic information may include demographic information including the gender and age of the examinee, basic health information including the weight, height and obesity of the examinee, disease-related information including the past history, drug history and family history of the examinee, and test information regarding the blood test, genetic test, vital signs including blood pressure and oxygen saturation, and biosignals of the examinee.

Furthermore, the single-lead electrocardiogram measurement unit may include a wearable electrocardiogram patch, a smartwatch, or an electrocardiogram bar.

Moreover, the prediction unit may further include a data conversion module configured to generate the asynchronous electrocardiogram data, measured from the single-lead electrocardiogram measurement unit, as numerical data through a specific formula; and the diagnostic algorithm may use numerical data corresponding to the asynchronous electrocardiogram data as input.

### Advantageous Effects

According to the present invention, there are provided advantageous effects in that 2- or more-lead asynchronous electrocardiograms may be easily measured utilizing a single-lead electrocardiogram device or a 6-lead electrocardiogram device, a disease may be predicted from the measured 2- or more-lead asynchronous electrocardiogram data, and multi-channel electrocardiogram data may be generated from the 2- or more-lead asynchronous electrocardiogram data, so that the presence/absence of a disease and the degree of the disease can be output and predicted with higher accuracy.

### Description of Drawings

FIG. 1 illustrates a block diagram of a system for predicting a health condition using a single-lead electrocardiogram device according to an embodiment of the present invention; and
FIG. 2 illustrates a flowchart of a prediction method performed by the system for predicting a health condition using a single-lead electrocardiogram device shown in FIG. 1.

### Mode for Invention

Embodiments of the present invention having the above-described features will be described in more detail below with reference to the accompanying drawings.

A system for predicting a health condition using a single-lead electrocardiogram device according to an embodiment of the present invention includes: a single-lead electrocardiogram measurement unit 110 provided with two electrodes, and configured to obtain asynchronous electrocardiogram data by measuring electrocardiograms for at least two electrical axes at different times; and a prediction unit 120 configured to predict the presence/absence of a disease and the degree of the disease from the asynchronous electrocardiogram data input from the single-lead electrocardiogram measurement unit 110 through a diagnostic algorithm 121 previously constructed by being trained on a plurality of standard electrocardiogram datasets in which standard lead electrocardiograms measured for the same electrical axes are matched to the presence/absence of diseases and degrees of the diseases corresponding to the standard lead electrocardiograms. Accordingly, the system for predicting a health condition using a single-lead electrocardiogram device predicts a disease from the 2- or more-lead asynchronous electrocardiogram data measured by a single-lead electrocardiogram device.

The system for predicting a health condition using a single-lead electrocardiogram device configured as described above will be described in more detail below with reference to the drawings as follows.

First, the single-lead electrocardiogram measurement unit 110 is provided with two electrodes, obtains asynchronous electrocardiogram data by measuring electrocardiograms for at least two electrical axes at different times in such a manner as to bring the two electrodes into contact with two portions of the body of an examinee, and transmits the obtained asynchronous electrocardiogram data to the prediction unit 120 through short-distance communication.

For example, a plurality of asynchronous electrocardiograms for at least two different electrical axes may be measured by measuring a lead-I electrocardiogram corresponding to one electrical axis in such a manner as to bring the respective electrodes into contact with both hands and also measuring a lead-II electrocardiogram corresponding to another electrical axis in such a manner as to bring the respective electrodes into contact with the right and left ankles, which are different from the previous body combination for contact with the electrodes.

Furthermore, the single-lead electrocardiogram measurement unit 110 may measure asynchronous or synchronous electrocardiograms by including a wearable electrocardiogram patch 111, a smartwatch 112, or a 6-lead electrocardiogram bar for short-term measurement capable of contact or contactless electrocardiogram measurement during daily life, and may predict a corresponding health condition by additionally generating a plurality of pieces of electrocardiogram data through an electrocardiogram data generation unit 130 and inputting the plurality of pieces of electrocardiogram data to the diagnostic algorithm 121 constructed using standard lead electrocardiogram data.

In this case, the single-lead electrocardiogram measurement unit 110 may measure successive electrocardiograms of an examinee and transmit them to the prediction unit 120, or may measure two electrocardiograms at different times and transmit them to the prediction unit 120.

Next, the prediction unit 120 is a component that predicts a health condition from the asynchronous electrocardiogram data of the single-lead electrocardiogram measurement unit 110 through the previously trained diagnostic algorithm 121. Through the diagnostic algorithm 121 previously constructed by being trained on a plurality of standard electrocardiogram datasets in which standard lead electrocardiograms measured for the same electrical axes as the electrocardiograms previously measured by the single-lead electrocardiogram measurement unit 110 and the presence/absence of diseases and the degrees of the diseases corresponding to the standard lead electrocardiograms are matched to each other, the prediction unit 120 determines a health condition by predicting the presence/absence of a disease and the degree of the disease from the asynchronous electrocardiogram data input from the single-lead electrocardiogram measurement unit 110.

In other words, the diagnostic algorithm 121 may be constructed to output and predict the presence/absence of a disease and degree of the disease corresponding to asynchronous standard lead electrocardiogram data by using the standard lead electrocardiogram data, measured asynchronously for any electrical axes, as input. Accordingly, the diagnostic algorithm 121 may predict a health condition from asynchronous electrocardiogram data transmitted from the single-lead electrocardiogram measurement unit 110. In this case, the standard lead electrocardiogram data may be divided into 2.5 second units and then stored, or may be stored asynchronously.

Alternatively, the diagnostic algorithm 121 may be constructed to output and predict the presence/absence of a disease and degree of the disease corresponding to standard lead electrocardiogram data by using the standard lead electrocardiogram data, in which time-series information has been deleted from standard lead electrocardiogram data measured synchronously for a plurality of electrical axes, as input. Accordingly, the diagnostic algorithm 121 may predict the health condition of the examinee from the asynchronous electrocardiogram data transmitted from the single-lead electrocardiogram measurement unit 110.

Alternatively, the diagnostic algorithm 121 may be constructed to convert a plurality of pieces of asynchronous electrocardiogram data, measured and input at different times from the single-lead electrocardiogram measurement unit 110, into synchronous electrocardiogram data and predict the presence/absence of a disease and the degree of the disease from the resulting synchronous electrocardiogram data.

Accordingly, the diagnostic algorithm 121 may match a plurality of pieces of asynchronous electrocardiogram data measured and input at different times by the single-lead electrocardiogram measurement unit 110 against standard lead electrocardiogram data which is measured asynchronously, against standard lead electrocardiogram data from which time-series information has been deleted, or against synchronous standard lead electrocardiogram data.

Furthermore, through the electrocardiogram data generation unit 130, based on a plurality of asynchronous electrocardiogram data measured for at least two electrical axes by the single-lead electrocardiogram measurement unit 110, a plurality of standard lead electrocardiogram data that are not measured by the single-lead electrocardiogram measurement unit 110 and, thus, are not matched to standard lead electrocardiograms are generated. Accordingly, the diagnostic algorithm 121 may output and predict the presence/absence of a disease and the degree of the disease by using the asynchronous electrocardiogram data measured by the single-lead electrocardiogram measurement unit 110 and the standard lead electrocardiogram data generated by the electrocardiogram data generation 130 as input or using the standard lead electrocardiogram data generated by the electrocardiogram data generation unit 130 as input.

In this case, when a plurality of pieces of standard lead electrocardiogram data are generated, the unique characteristics of an electrocardiogram for each lead are determined, so that the asynchronous electrocardiogram data input from the single-lead electrocardiogram measurement unit 110 is matched to corresponding specific standard lead electrocardiogram data, and the remaining standard lead electrocardiogram data not matched to the specific standard lead electrocardiogram data is generated, with the result that a plurality of pieces of new standard lead electrocardiogram data can be generated.

Alternatively, the standard lead electrocardiogram data generated by the electrocardiogram data generation unit 130 may be asynchronous or synchronous electrocardiograms. As described above, when the diagnostic algorithm 121 uses standard lead electrocardiogram data which is measured asynchronously or standard lead electrocardiogram data from which time-series information has been deleted, asynchronous electrocardiogram data may be generated or electrocardiogram data may be generated without taking into consideration synchronization.

Through this, the prediction of the presence/absence of a disease and the diagnosis of the degree of the disease that need to be diagnosed using a multi-lead electrocardiogram are performed using the asynchronous electrocardiograms measured by the single-lead electrocardiogram measurement unit 110. Accordingly, a disease is analyzed more accurately based on a plurality of pieces of electrocardiogram information, so that the measurement, diagnosis, examination, and prediction of a health condition can be performed.

For example, in the case of a disease that can be diagnosed using a single-lead electrocardiogram such as arrhythmia, electrocardiograms for each beat are generated for various electrical axes by using a 2-lead electrocardiogram and a plurality of electrocardiograms generated based on it, so that the more accurate measurement, diagnosis, examination, and prediction of a health condition can be performed. In the case of a disease that can be diagnosed using a multi-lead electrocardiogram such as myocardial infarction, myocardial infarction may be diagnosed by additionally generating a plurality of electrocardiograms.

Alternatively, although the diagnostic algorithm 121 may be a model that performs the measurement, diagnosis, examination, and prediction of a health condition using only electrocardiogram data corresponding to 2 leads to be used in standard lead electrocardiogram data for the diagnosis of a disease, it is not limited to 2 leads, but additionally generated lead electrocardiogram data may also be used, as described above.

In other words, the electrocardiogram data accumulated in most medical institutions is standard 12-lead electrocardiogram data. A standard 12-lead electrocardiogram is generated from a 2-lead electrocardiogram through the electrocardiogram data generation unit 130, and the electrocardiogram generated in this manner is input to an algorithm that performs the measurement, diagnosis, examination, and prediction of a health condition based on the standard 12-lead electrocardiogram data of the medical institutions, so that more accurate prediction results can be output and a wider range of a health condition can be predicted utilizing the 2-lead electrocardiogram.

As an example, in the case of measurement performed by the single-lead electrocardiogram measurement unit 110, when electrocardiogram data contains a lot of noise in electrocardiogram data for one or more leads or sections or electrode contact is lost and, thus, measurement is not performed appropriately, the more accurate measurement, diagnosis, examination, and prediction of a health condition may be performed by generating a noise-free electrocardiogram and filling in the missing electrocardiogram data through the electrocardiogram data generation unit 130.

Furthermore, through the single-lead electrocardiogram measurement unit 110 and the prediction unit 120, a health condition is monitored by measuring the reference electrocardiogram of an examinee in his or her usual healthy condition, and whether the electrocardiogram was measured without error and whether the health condition of the examinee is normal are predicted by comparing the electrocardiogram measured and input in real time from the single-lead electrocardiogram measurement unit 110 during daily life with the reference electrocardiogram. Furthermore, when an error or abnormality is predicted, warning information may be generated through a warning unit 140 and transmitted together with a beep sound through the single-lead electrocardiogram measurement unit 110 in the form of a smartwatch or through a separate smart device.

More specifically, at the beginning of use, the reference electrocardiogram measured by the single-lead electrocardiogram measurement unit 110 is stored and then additional lead electrocardiograms are generated, so that a 12-lead electrocardiogram can be continuously monitored. A lead II electrocardiogram is measured by bringing the smartwatch into contact with the stomach while holding the smartwatch 112 in the right hand and is then stored as a reference electrocardiogram. In normal times, a lead I electrocardiogram is measured by making contact using the right hand while wearing the smartwatch 112 on the left hand, and at the same time, a plurality of lead electrocardiograms, including a lead II electrocardiogram, are generated using the lead I electrocardiogram. Accordingly, using the smartwatch 112, the measurement, diagnosis, examination, and prediction of various health conditions may be performed.

Alternatively, before the wearable electrocardiogram patch 111 is attached, a lead I electrocardiogram is measured by holding the wearable electrocardiogram patch 111 in both hands and is then stored as a reference electrocardiogram. Thereafter, a lead V electrocardiogram is measured by attaching the wearable electrocardiogram patch 111, and a multi-lead synchronized electrocardiogram is generated based on the continuously measured or monitored lead V electrocardiogram. Accordingly, the more accurate measurement, diagnosis, examination, and prediction of a health condition may be performed.

Furthermore, the diagnostic algorithm 121 is trained with the individual characteristic information of an examinee reflected therein, and the prediction unit 120 may predict the presence/absence of a disease and the degree of the disease from the asynchronous electrocardiogram data measured by the single-lead electrocardiogram measurement unit 110 with the individual characteristic information reflected therein.

For example, when the standard lead electrocardiogram data that is used when the diagnostic algorithm 121 is trained is applied, the presence/absence of a corresponding disease and the degree of the disease may be predicted with individual characteristic information reflected therein. Alternatively, standard lead electrocardiogram data is generated by the electrocardiogram data generation unit 130 with the individual characteristic information of an examinee whose electrocardiogram is measured by the single-lead electrocardiogram measurement unit 110 reflected therein, and then the presence/absence of a corresponding disease and the degree of the disease may be predicted.

In this case, the individual characteristic information may include demographic information including the gender and age of the examinee, basic health information including the weight, height and obesity of the examinee, disease-related information including the past history, drug history and family history of the examinee, and test information regarding a blood test, genetic test, vital signs including blood pressure and oxygen saturation, and biosignals of the examinee.

Meanwhile, the prediction unit 120 may use not only a graphed electrocardiogram but also a digitized electrocardiogram as input. For example, the prediction unit 120 may further include a data conversion module 122 configured to convert asynchronous electrocardiogram data, measured by the single-lead electrocardiogram measurement unit 110, into numerical data corresponding to an electrocardiogram through a specific formula. The diagnostic algorithm 121 may predict a health condition by using numerical data corresponding to asynchronous electrocardiogram data as input.

Furthermore, the diagnostic algorithm 121 may be constructed as a variety of deep learning models such as a convolutional neural network, LSTM, an RNN, and an MLP, and may be constructed as various machine learning models such as logistic regression, a principlebased model, random forests, and a support vector machine.

For example, the diagnostic algorithm 121 may be constructed as a deep learning model using two or more pieces of electrocardiogram data measured at different times with a time difference therebetween. At least two electrocardiograms may be integrated into and input as one piece of electrocardiogram data or two or more electrocardiograms may be input to a deep learning model, the semantic features, that is, spatial time series features, measured in the midst thereof may be extracted, and then, based on this, the measurement, diagnosis, examination, and prediction of a health condition at the time when the measurement is made by the single-lead electrocardiogram measurement unit 110 or a future health condition may be performed by comparing the two electrocardiograms.

In this case, according to a method of mixing electrocardiograms measured at two or more times, a single electrocardiogram may be divided into beats, and then beats for the same lead measured at different times may be paired and input or the semantic features or result values extracted after input may be compared to each other.

Alternatively, rather than dividing an electrocardiogram measured at two or more times into beats, electrocardiogram data itself may be merged together. The electrocardiogram data measured at two or more times may be merged together and then input. The electrocardiogram data may be separated and merged for each lead and then input. The electrocardiograms measured at different times may be input to the deep learning layer of a deep learning model, semantic features or output values may be extracted, and then these may be merged to output a final conclusion. The electrocardiograms measured at different times may be separated for each lead and then input to the deep learning layer, and the features or output values extracted through deep learning may be merged at the back end to output the final conclusion.

In this case, when the electrocardiograms measured at two or more times are mixed and then used, the electrocardiograms may be input in an asynchronous state, may be synchronized on a per-bit basis, or may be synchronized based on deep learning, merged and then used.

In other words, a deep learning model may be constructed utilizing standard lead electrocardiogram data. There is developed a deep learning model that measures, diagnoses, examines, and predicts a health condition through the input of one electrocardiogram at each time. Thereafter, using the corresponding deep learning model, the electrocardiograms measured at two or more times are input to the deep learning model. Thereafter, results may be predicted by combining the semantic features or final output values output from the deep learning model.

As described above, as a method of combining the final output values, a variety of deep learning methods such as a convolutional neural network, LSTM, an RNN, and an MLP may be used, and various machine learning methods such as logistic regression, a rule-based model, random forests, and a support vector machine may also be used.

Through the prediction unit 120 described above, diseases of the circulatory system, endocrine, nutritional and metabolic diseases, neoplastic diseases, mental and behavioral disorders, diseases of the nervous system, diseases of the eyes and appendages, diseases of the ears and mastoids, diseases of the respiratory system, diseases of the digestive system, diseases of the skin and skin tissue, diseases of the musculoskeletal system and compressive tissue, diseases of the genitourinary system, pregnancy, childbirth and postpartum diseases, congenital anomalies, deformities, and chromosomal abnormalities may be diagnosed and predicted.

In addition, through the prediction unit 120, the damage caused by physical trauma may be identified, the prognosis thereof may be checked and the pain thereof may be measured, the risk of death or worsening attributable to trauma may be predicted, concurrent complications may be detected or predicted, and specific conditions that appear before or after birth may also be identified.

In addition, through the prediction unit 120, for the healthcare field, the health condition of an examinee, which can lead to services such as aging, sleep, weight, blood pressure, blood sugar, oxygen saturation, metabolism, stress, tension, fear, drinking, smoking, problem behavior, lung capacity, exercise volume, pain management, obesity, body mass, body composition, diet, type of exercise, lifestyle pattern recommendations, emergency management, chronic disease management, medication prescription, test recommendation, checkup recommendation, nursing, telehealth management, telemedicine, vaccination, and post-vaccination management, may be measured, diagnosed, examined, and predicted.

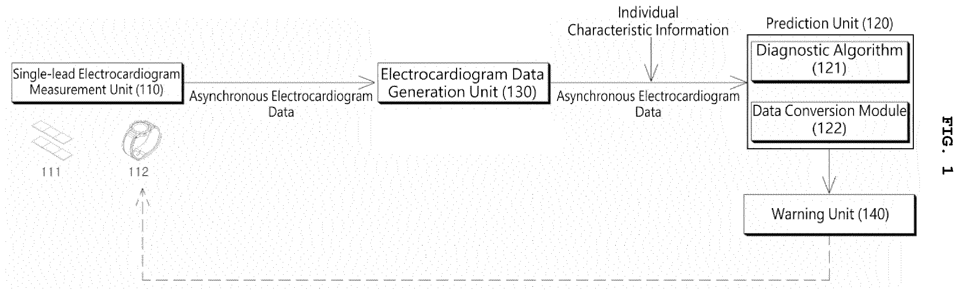

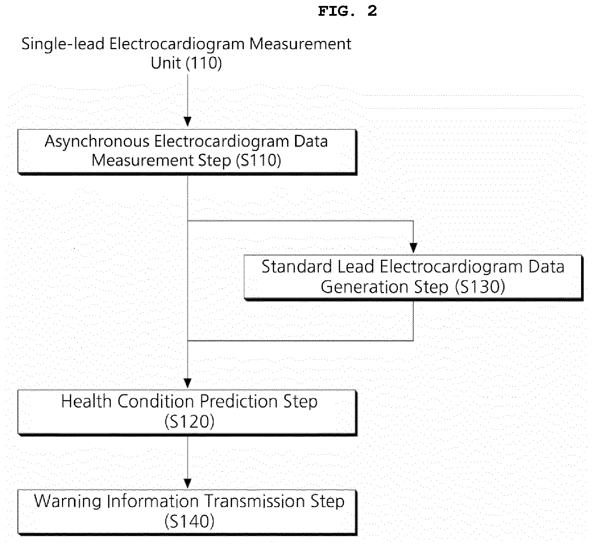

FIG. 2 illustrates a flowchart of a prediction method performed by the system for predicting a health condition using a single-lead electrocardiogram device shown in FIG. 1. The prediction method will be described in detail with reference to this drawing as follows.

First, asynchronous electrocardiogram data is obtained by measuring electrocardiograms for at least two electrical axes at different times through the single-lead electrocardiogram measurement unit 110 provided with two electrodes in such a manner as to provide two electrodes and bring the two electrodes into contact with two portions of the body of an examinee, and is then transmitted to the prediction unit 120 through short-distance communication in step S110.

Thereafter, there is performed the step of predicting a health condition from the asynchronous electrocardiogram data of the single-lead electrocardiogram measurement unit 110 via the previously trained diagnostic algorithm 121 through the prediction unit 120. Through the diagnostic algorithm 121 previously constructed by being trained on a plurality of standard electrocardiogram datasets in which standard lead electrocardiograms measured for the same electrical axes as the electrocardiograms previously measured by the single-lead electrocardiogram measurement unit 110 are matched to the presence/absence of diseases and degrees of the diseases corresponding to the standard lead electrocardiograms, a health condition may be determined by predicting the presence/absence of a disease and the degree of the disease from the asynchronous electrocardiogram data input from the single-lead electrocardiogram measurement unit 110.

Meanwhile, through the electrocardiogram data generation unit 130, based on the plurality of pieces of asynchronous electrocardiogram data measured for the at least two electrical axes by the single-lead electrocardiogram measurement unit 110, a plurality of pieces of standard lead electrocardiogram data that are not measured by the single-lead electrocardiogram measurement unit 110 and, thus, are not matched to standard lead electrocardiograms are generated in step S130. Accordingly, the diagnostic algorithm 121 may output and predict the presence/absence of a disease and the degree of the disease by using the asynchronous electrocardiogram data measured by the single-lead electrocardiogram measurement unit 110 and the standard lead electrocardiogram data generated by the electrocardiogram data generation unit 130 as input or using the standard lead electrocardiogram data generated by the electrocardiogram data generation unit 130 as input.

Thereafter, by means of the warning unit 140, through the single-lead electrocardiogram measurement unit 110 and the prediction unit 120, a health condition is monitored by measuring the reference electrocardiogram of an examinee in his or her usual healthy condition, and whether the electrocardiogram was measured without error and whether the health condition of the examinee is normal are predicted by comparing the electrocardiogram, measured and input in real time from the single-lead electrocardiogram measurement unit 110 during daily life, with the reference electrocardiogram. Furthermore, when an error or abnormality is predicted, warning information may be generated and transmitted together with a beep sound through the single-lead electrocardiogram measurement unit 110 in the form of a smartwatch or through a separate smart device.

Therefore, through the configuration of the system for predicting a health condition using a single-lead electrocardiogram device described above, 2- or more-lead asynchronous electrocardiograms may be easily measured utilizing a single-lead electrocardiogram device or a 6-lead electrocardiogram device, a disease may be predicted from the measured 2- or more-lead asynchronous electrocardiogram data, and multi-channel electrocardiogram data may be generated from the 2- or more-lead asynchronous electrocardiogram data, so that the presence/absence of a disease and the degree of the disease can be output and predicted with higher accuracy.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or the configurations.

## Claims

1. A system for predicting a health condition using a single-lead electrocardiogram device, the system comprising:
a single-lead electrocardiogram measurement unit provided with two electrodes, and configured to obtain asynchronous electrocardiogram data by measuring electrocardiograms for at least two electrical axes at different times; and
a prediction unit configured to predict presence/absence of a disease and a degree of the disease from the asynchronous electrocardiogram data input from the single-lead electrocardiogram measurement unit based on a diagnostic algorithm previously constructed by being trained on a plurality of standard electrocardiogram datasets in which standard lead electrocardiograms measured for same electrical axes are matched to presence/absence of diseases and degrees of the diseases corresponding to the standard lead electrocardiograms.

2. The system of claim 1, wherein the diagnostic algorithm is constructed to output and predict presence/absence of a disease and a degree of the disease corresponding to asynchronously measured standard lead electrocardiogram data by using the asynchronously measured standard lead electrocardiogram data as input.

3. The system of claim 1, wherein the diagnostic algorithm is constructed to output and predict presence/absence of a disease and a degree of the disease corresponding to standard lead electrocardiogram data by using the standard lead electrocardiogram data, in which time series information has been deleted from synchronously measured standard lead electrocardiogram data, as input.

4. The system of claim 1, wherein the diagnostic algorithm is constructed to convert a plurality of pieces of asynchronous electrocardiogram data, measured and input at different times from the single-lead electrocardiogram measurement unit, into synchronous electrocardiogram data and predict presence/absence of a disease and a degree of the disease from the resulting synchronous electrocardiogram data.

5. The system of claim 4, further comprising an electrocardiogram data generation unit configured to generate a plurality of pieces of standard lead electrocardiogram data that are not measured by the single-lead electrocardiogram measurement unit and, thus, are not matched to standard lead electrocardiograms based on a plurality of pieces of asynchronous electrocardiogram data measured by the single-lead electrocardiogram measurement unit;
wherein the diagnostic algorithm outputs and predicts presence/absence of a disease and a degree of the disease by using the measured asynchronous electrocardiogram data and the generated standard lead electrocardiogram data as input or using the generated standard lead electrocardiogram data as input.

6. The system of claim 1, wherein:
the diagnostic algorithm is trained with individual characteristic information of an examinee reflected therein; and
the prediction unit predicts presence/absence of a disease and a degree of the disease from the asynchronous electrocardiogram data, measured by the single-lead electrocardiogram measurement unit, with the individual characteristic information reflected therein.

7. The system of claim 6, wherein the individual characteristic information includes demographic information including gender and age of the examinee, basic health information including weight, height and obesity of the examinee, disease-related information including a past history, drug history and family history of the examinee, and test information regarding a blood test, genetic test, vital signs including blood pressure and oxygen saturation, and biosignals of the examinee.

8. The system of claim 1, wherein the single-lead electrocardiogram measurement unit includes a wearable electrocardiogram patch, a smartwatch, or an electrocardiogram bar.

9. The system of claim 1, wherein:
the prediction unit further includes a data conversion module configured to generate the asynchronous electrocardiogram data, measured from the single-lead electrocardiogram measurement unit, as numerical data through a specific formula; and
the diagnostic algorithm uses numerical data corresponding to the asynchronous electrocardiogram data as input.
